# DEMANDE DE BREVET EUROPEEN

(11) **EP 4 797 056 A1**
(43) Date de publication de la demande: **26.08.2026**
(21) Numéro de dépôt: 26157104.6
(22) Date de dépôt: 06.02.2026
(51) Int. Cl.: G06F 3/01, A61B 5/372, A61F 2/72, A61F 4/00

(54) **DISPOSITIF ÉLECTRONIQUE COMPRENANT UN EXTRACTEUR DE CARACTÉRISTIQUES ET PROCÉDÉ D EXTRACTION DE CARACTÉRISTIQUES CORRESPONDANT**

(30) Priorité: 19.02.2025 FR 2501705
(71) Demandeur: Commissariat à l'Energie Atomique et aux Energies Alternatives, 75015 Paris (FR)
(72) Inventeur: SAAD, Joe, 38054 Grenoble Cedex 9 (FR); EVANS, Adrian, 38054 Grenoble Cedex 9 (FR); MIRO PANADES, Ivan, 38054 Grenoble Cedex 9 (FR)
(74) Mandataire: Germain Maureau

(57) **Abrégé**

La présente invention concernant un dispositif électronique (1) comprenant un capteur primaire (2) configuré pour réaliser l'acquisition d'un signal de mesure (x) d'au moins un paramètre physiologique d'un être vivant ; un extracteur de caractéristique (3) ; un décodeur primaire (4) utilisant la caractéristique extraite (S) et configuré pour déterminer un signal de commande (C) d'un actionneur (6) avec lequel l'être vivant interagit. L'extracteur de caractéristique (3) est configuré pour réaliser l'extraction de caractéristiques (S) du signal de mesure (x) de façon approximée selon plusieurs niveaux de précision en fonction d'un paramètre de contrôle de précision (PCP) ajusté en fonction d'une valeur d'un paramètre d'évaluation de qualité (PEQ). La présente invention concerne également un procédé d'extraction de caractéristiques.

## Description

### Domaine technique

Le domaine technique de l'invention concerne les interfaces neuronales directes, usuellement désignées par le terme « BCI » (Brain Computer Interface), destinées à commander, à partir de signaux neurophysiologiques, un actionneur, mais également tout autre système embarqué ou implantable mettant en œuvre une chaîne de traitement comprenant une étape d'extraction de caractéristiques.

### Etat de la technique

Les interfaces neuronales directes permettent à des utilisateurs de commander des actionneurs par la pensée. Ces interfaces peuvent être destinées à des personnes en situation de handicap ou à d'autres utilisateurs. A cet effet, les signaux électrophysiologiques émis par le cortex sont détectés et enregistrés, puis traités ou décodés par des algorithmes permettant de former un signal de commande, pour commander des actionneurs. Le signal de commande peut permettre le pilotage d'un exosquelette, d'un ordinateur d'un robot, pour fournir une assistance à l'utilisateur, le décodage de paroles, ou encore une stimulation des muscles de l'utilisateur.

Les algorithmes mis en œuvre permettent une traduction d'une instruction donnée par l'utilisateur, cette instruction étant captée, par des électrodes, sous la forme des signaux dits électrophysiologiques, car représentatifs de l'activité électrique des neurones. Cette activité électrique peut être au niveau du cortex, au moyen d'électrodes corticales disposées dans la boite crânienne. Elle peut également être mesurée par des électrodes d'électroencéphalographie, moins intrusives car disposées sur le scalp, mais également moins performantes, notamment au niveau de la résolution spatiale. Une autre solution est d'enregistrer les signaux électrophysiologiques par magnétoencéphalographie, ce qui nécessite une installation dédiée.

Les algorithmes mis en oeuvre sont généralement basés sur un modèle prédictif.

Le modèle prédictif utilise des données d'entrée, obtenues par un prétraitement des signaux électrophysiologiques enregistrés visant à réaliser une extraction de caractéristiques, pour établir un signal de commande, à destination de l'actionneur ou des actionneurs.

Les données d'observation ou caractéristiques extraites sont généralement multidimensionnelles, et peuvent comprendre par exemple :
- une composante spatiale, représentative de l'origine spatiale du signal électrophysiologique ;
- une composante fréquentielle, représentative de l'intensité du signal électrophysiologique dans différentes bandes de fréquence ;
- une composante temporelle qui correspond à un échantillon temporel du signal électrophysiologique dans lequel l'analyse fréquentielle est effectuée.

Les données d'observation ou caractéristiques extraites peuvent aussi refléter d'autres informations comme par exemple des déphasages entre les signaux, des corrélations, des propriétés intrinsèques à un signal, comme la variance ou des paramètres chaotiques.

Chaque donnée d'observation est associée à une époque, c'est-à-dire à un échantillon temporel de durée prédéterminée, après que l'utilisateur ait eu l'intention d'effectuer la tâche. Le terme époque correspond à l'équivalent du terme anglo-saxon « epoch ». A chaque époque, on forme un tenseur d'observation, rassemblant les données d'observation. A partir du tenseur d'observation, on alimente un modèle prédictif. Le modèle prédictif, appliqué au tenseur d'observation, permet d'estimer un signal de commande, permettant la commande des actionneurs. Le signal de commande est généralement exprimé par un vecteur de commande.

L'un des principaux défis dans le développement des BCls, mais également dans de nombreux autres systèmes portables ou implantables utilisant une extraction de caractéristiques réside dans l'optimisation de la consommation d'énergie, particulièrement pour les applications portables et implantables. En effet, la chaine de traitement comprenant l'extraction des caractéristiques, le décodage et la génération d'un signal de commande est très énergivore, l'extraction de caractéristiques étant une partie particulièrement énergivore de cette chaine.

Des solutions ont été proposées pour limiter la consommation énergétique, par exemple dans le document U. Shin et al., "NeuralTree: A 256-Channel 0.227-uJ/Class Versatile Neural Activity Classification and Closed-Loop Neuromodulation SoC", Nov. 2022, doi: 10.1109/JSSC.2022.3204508. Ce document présente un système sur puce (SoC) pour la classification des activités neuronales et la neuromodulation en boucle fermée. Dans ce système, le choix des caractéristiques extraites est fait une fois au moment de l'apprentissage du modèle, lors de la phase d'entraînement. Un arbre de décision est construit, et à chaque nœud de l'arbre, un sous-ensemble de caractéristiques est utilisé. Le choix des caractéristiques extraites, à chaque nœud, est fait de manière à minimiser l'énergie. Ce système présente donc une approche statique prédéterminée lors de l'apprentissage qui définit de façon binaire l'utilisation ou la non utilisation d'une caractéristique et ne permet pas une adaptation dynamique aux conditions d'utilisation du système.

La présente invention a pour objet de résoudre tout ou une partie des inconvénients mentionnés ci-dessus.

### Exposé de l'invention

A cet effet, la présente invention concerne un dispositif électronique comprenant :
- Un capteur primaire configuré pour réaliser l'acquisition d'un signal de mesure d'au moins un paramètre physiologique d'un être vivant ;
- Un extracteur de caractéristique configuré pour extraire une caractéristique du signal de mesure ;
- Un décodeur primaire utilisant la caractéristique extraite et configuré pour déterminer un signal de commande ;
- Un actionneur avec lequel l'être vivant interagit ;
- Un module de pilotage de l'actionneur configuré pour piloter l'actionneur en fonction du signal de commande déterminé par le décodeur primaire.

L'extracteur de caractéristique est configuré pour pouvoir réaliser l'extraction de caractéristiques du signal de mesure de façon approximée selon plusieurs niveaux de précision, le niveau de précision requis étant fonction d'un paramètre de contrôle de précision souhaité, la consommation électrique de l'extracteur de caractéristique augmentant avec le niveau de précision ;
Le dispositif comprend en outre un module de contrôle configuré pour déterminer un paramètre d'évaluation de qualité de l'action de l'actionneur en interaction avec ledit être vivant ;
Le module de contrôle est configuré pour ajuster le paramètre de contrôle de précision en fonction d'une valeur du paramètre d'évaluation de qualité.

Selon une possibilité, ledit paramètre de contrôle de précision choisi correspond au niveau de précision le plus faible permettant de faire tendre la valeur du paramètre d'évaluation de qualité vers une gamme de valeurs de qualité prédéfinie ou de maintenir la valeur du paramètre d'évaluation de qualité dans une gamme de valeurs de qualité prédéfinie.

Grâce aux dispositions selon l'invention, un contrôle dynamique de la qualité des caractéristiques extraites des signaux de mesure est réalisé avec une boucle de rétroaction, ce qui permet un ajustement continu afin de réduire la complexité des calculs effectués quand des caractéristiques de haute qualité ne sont pas nécessaires et ainsi de réduire la consommation globale du dispositif. Cette approche est applicable lorsque l'erreur de calcul occasionnée a peu d'influence sur la sortie du système. Cette approche vise à maximiser l'efficacité énergétique tout en maintenant un fonctionnement acceptable du système complet, ouvrant ainsi la voie à des applications plus portables et moins énergivores.

Cette approche permet une adaptation de la qualité des caractéristiques extraites, en fonction des exigences de la tâche. Par exemple, pour des tâches simples nécessitant une précision moindre, le dispositif selon l'invention peut réduire la qualité des caractéristiques extraites, ce qui permet de réaliser des économies d'énergie significatives. Pour des tâches plus complexes nécessitant une précision élevée, le dispositif peut augmenter la qualité des caractéristiques extraites, assurant ainsi une performance suffisante.

La modification de la qualité, peut être faite en cours de fonctionnement du circuit. Il n'est pas nécessaire de l'arrêter pour la changer. Le fonctionnement des autres éléments de la chaine de traitement n'est pas affecté.

Selon différentes possibilités, les niveaux de précisions peuvent être des niveaux de précision continus ou discret.

On entend par niveau de précision le niveau de précision des méthodes de calcul ou en d'autre termes le niveau d'approximation des méthodes par rapport à un calcul en pleine précision ou calcul exact.

On entend par « faire tendre la valeur du paramètre d'évaluation de qualité vers une gamme de valeurs de qualité prédéfinie ou de maintenir la valeur du paramètre d'évaluation de qualité dans une gamme de valeurs de qualité prédéfinie » que l'ajustement du paramètre de contrôle de précision vise à ce que le paramètre d'évaluation de qualité soit inclus dans la gamme de valeur de qualité. Grâce à ces dispositions, une régulation du paramètre de contrôle de précision en fonction du paramètre d'évaluation de qualité est obtenue. Le choix de la valeur du paramètre de contrôle de qualité peut être réalisé de façon itérative. L'objectif de maintenir dans la gamme de valeur de qualité peut conduire à ce que le paramètre d'évaluation de la qualité sorte ponctuellement de cette gamme de valeur lors de la régulation, notamment en vue de minimiser la valeur du paramètre de contrôle de la qualité.

Selon une possibilité, le paramètre de contrôle de précision est calculé comme fonction continue ou discrète du paramètre d'évaluation de qualité.

Selon une possibilité, l'extracteur de caractéristique peut réaliser l'extraction de caractéristique du signal de mesure ou en pleine précision selon un niveau de précision, l'extraction en pleine précision correspondant à un calcul exact.

Selon une possibilité, la gamme de valeurs de qualité est définie par un écart déterminé entre le paramètre d'évaluation de la qualité courant et le paramètre d'évaluation de qualité qui serait obtenu avec un calcul en pleine précision.

Selon une possibilité, le signal de mesure est un signal électrophysiologique, en particulier un signal électrophysiologique émis par le cortex.

Selon une possibilité, le dispositif comprend un circuit intégré qui comprend au moins l'un parmi l'extracteur de caractéristiques, le module de contrôle, et le décodeur primaire.

Selon une possibilité, l'actionneur est un élément parmi un stimulateur de moelle épinière, un stimulateur musculaire, un actionneur d'exosquelette, un générateur de parole, un actionneur de fauteuil roulant, tel qu'un moteur de fauteuil roulant, un afficheur.

Selon une possibilité, l'extracteur de caractéristique est configuré pour réaliser un calcul approximé d'une convolution entre le signal d'entrée et un signal de référence, en prenant en compte le paramètre de contrôle de précision.

Selon différentes possibilités, le signal de référence peut être une ondelette, une fonction trigonométrique, des coefficients de filtre, un signal permettant de calculer des coefficients de Fourier.

Cette technique permet d'extraire une information spectro-temporelle.

Selon une possibilité, le calcul approximé utilise une technique d'approximation du type transformée en ondelettes continues, linéaires par morceaux.

Selon une autre possibilité, le calcul approximé utilise une technique d'approximation du type transformée en ondelette quantifiée.

Selon une autre possibilité, le calcul approximé utilise une transformée en cosinus discrète.

Selon une autre possibilité, le calcul approximé utilise une transformée en ondelette continue avec un niveau de quantification adaptatif.

Selon une autre possibilité, le calcul approximé utilise une transformée de Fourier rapide avec résolution adaptative.

Selon une autre possibilité, le calcul approximé utilise des filtres numériques avec des supports de taille variable.

Selon une autre possibilité, le calcul approximé utilise un ajustement de la précision numérique, notamment du nombre de bits,

Selon une autre possibilité, une arithmétique approximative ou d'autres approches de calcul approximatifs peuvent être utilisés en adaptant le niveau d'approximation à une valeur du paramètre de contrôle de précision.

Selon une autre possibilité, le paramètre de contrôle de précision correspond à un niveau de précision d'une fonction d'approximation du signal de référence.

Selon une possibilité, le niveau de précision correspond à nombre de points utilisés pour approximer une ondelette.

Selon une possibilité, l'extracteur de caractéristique est configuré pour extraire un ensemble de caractéristiques du signal de mesure, le module de contrôle étant configuré pour déterminer un paramètre de contrôle de précision unique pour plusieurs caractéristiques extraites de l'ensemble de caractéristiques, ou pour déterminer un paramètre de contrôle de précision individuel pour chacune des caractéristiques extraites de l'ensemble de caractéristiques.

Selon une possibilité, le module de contrôle est configuré pour déterminer un paramètre d'évaluation de qualité unique pour plusieurs caractéristiques extraites de l'ensemble de caractéristiques, ou pour déterminer un paramètre d'évaluation de qualité individuel pour chacune des caractéristiques extraites de l'ensemble de caractéristiques.

Selon une possibilité, le module de contrôle peut être configuré pour ajuster le paramètre de contrôle de précision pour plusieurs caractéristiques en fonction d'un même paramètre d'évaluation de qualité.

Selon une possibilité, l'ensemble de caractéristiques extraites correspond à une pluralité de fréquences et/ou une pluralité de localisation spatiales.

La pluralité de caractéristiques extraites peut correspondre à une pluralité de fréquences et/ou une pluralité de localisation spatiales, correspondant par exemple au positionnement de plusieurs capteurs primaires ou sous-partie du capteur primaire. Plusieurs caractéristiques extraites peuvent aussi correspondre à des instants spécifiques.

Selon un mode de réalisation, le dispositif électronique comprend un décodeur secondaire configuré pour fournir un signal de satisfaction d'un utilisateur, le paramètre d'évaluation de qualité prenant en compte le signal de satisfaction de l'utilisateur.

Selon un mode de réalisation, le décodeur secondaire de niveau de satisfaction d'un utilisateur est configuré pour réaliser un décodage de caractéristiques extraites de signaux électrophysiologique émis par le cortex.

Selon une autre possibilité, le décodeur secondaire est configuré pour un réaliser un décodage sur la base des signaux d'entrée x, ou de caractéristiques simples à extraire sur la base de ces signaux, par exemple la moyenne et/ou la variance de ces signaux.

Le décodage peut être réalisé en temps réel.

Selon un mode de réalisation, le paramètre d'évaluation de qualité est le signal de satisfaction de l'utilisateur

Selon une possibilité, l'état de satisfaction de l'utilisateur est décodé quand il exécute une tâche. Si, lors du décodage, le décodeur identifie que l'utilisateur n'est pas satisfait, le module de contrôle est configuré pour incrémenter progressivement le paramètre de contrôle de précision et ainsi la précision des calculs afin de réduire l'erreur de décodage.

Ainsi, ce signal peut être utilisé pour contrôler dynamiquement la qualité des caractéristiques extraites

Selon un mode de réalisation, le module de contrôle est configuré pour réaliser ou commander périodiquement un calcul d'extraction de caractéristique exact et pour mesurer un écart entre le résultat obtenu avec une extraction de caractéristique exacte et celui obtenu avec une extraction de caractéristique approximée avec le niveau du paramètre de contrôle de précision d'extraction courant de façon à déterminer une valeur du paramètre d'évaluation de qualité.

Selon une possibilité, l'écart peut être évalué par une corrélation des caractéristiques extraites exactes et approximées.

Selon une autre possibilité, l'écart peut être évalué en comparant le résultat du décodage réalisé, par exemple en identifiant si le résultat du décodage est le même avec les caractéristiques extraites approximée et des caractéristiques extraites exactes, notamment sur la détermination d'un signal de commande ou d'une classe en sortie.

Selon un mode de réalisation, le module de contrôle est configuré pour déterminer le paramètre d'évaluation de qualité en prenant en compte une valeur d'un indicateur de performance, le dispositif comprenant un capteur de mesure secondaire configuré pour acquérir une grandeur de contrôle ou une interface utilisateur configurée pour collecter une information de contrôle provenant d'un utilisateur, le module de contrôle étant configuré pour déterminer le paramètre d'évaluation de qualité en prenant en compte l'indicateur de performance déterminé à partir d'une valeur de la grandeur de contrôle ou de l'information de contrôle.

Selon ces dispositions, l'indicateur de performance correspond à une « vérité terrain » objective correspondant à une mesure de capteur ou à un retour de l'utilisateur.

Selon différents exemples, un indicateur de performance peut être un taux de réussite des décodages, une précision des actions effectuées ou la variabilité des valeurs obtenus lors du décodage.

Selon une possibilité, si la performance diminue, le module de contrôle peut augmenter la qualité des caractéristiques extraites pour améliorer la précision.

Selon un mode de réalisation, le capteur de mesure secondaire est un capteur de position, ou une pluralité de capteurs de position configuré pour identifier des positions anormales d'éléments contrôlés par l'actionneur.

Selon un mode de réalisation, le capteur de mesure secondaire est un capteur de mouvement, ou une pluralité de capteurs de mouvement configuré(s) pour analyser le mouvement des effecteurs contrôlé par les actionneurs, comme l'exosquelette, ou les membres de l'utilisateur et discriminer des écarts à des scénarios connus tels que la marche, la course, ou la saisie d'objet.

Selon un mode de réalisation, l'interface utilisateur est un capteur d'interface utilisateur, comme par exemple un rhéostat similaire à un bouton de volume, qui permettrait à l'utilisateur d'ajuster de façon directe la qualité en fonction de son ressenti.

Selon un mode de réalisation, le module de contrôle est configuré pour déterminer le paramètre d'évaluation de qualité en prenant en compte une détection d'anomalie prenant en compte la valeur du signal de commande et la valeur des caractéristiques extraites.

Selon un mode de réalisation, l'analyse des erreurs est réalisée par bande de fréquence ou par caractéristique extraite. Ainsi, le module de contrôle est configuré pour identifier les fréquences ou les caractéristiques, impliquées dans une tâche donnée, qui nécessitent une amélioration. Selon une possibilité, le module de contrôle peut augmenter la qualité des caractéristiques extraites pour les fréquences spécifiques identifiées en ajustant le paramètre de contrôle de précision.

Selon un mode de réalisation, la détection d'anomalie peut se baser sur la comparaison entre les valeurs ou amplitudes des caractéristiques extraites et une référence de valeurs attendues.

Selon un mode de réalisation, le dispositif électronique comprend une base de référence comprenant les références de valeurs attendues pour les valeurs des caractéristiques extraites en lien avec des valeurs du signal de commande.

Selon un mode de réalisation, le module de contrôle est configuré pour déterminer le paramètre d'évaluation de qualité en prenant en compte une valeur d'un niveau de confiance ou de probabilité d'un résultat du décodeur primaire.

Ces dispositions permettent d'utiliser les probabilités fournies par le décodeur primaire comme niveau de confiance en la décision émise. Par exemple, des décodeurs comme des classificateurs linéaires ou des arbres de décision associent une probabilité à une classe majoritaire choisie. Cette probabilité pourrait être utilisée pour contrôler le niveau de qualité des caractéristiques extraites.

Selon une possibilité, le module de contrôle peut être configuré pour associer le niveau de confiance ou probabilité d'une classe décodée à un niveau de qualité nécessaire au décodage suivant, et ainsi, déterminer à chaque étape le paramètre de contrôle de précision des caractéristiques extraites calculées.

Selon une autre possibilité, le module de contrôle peut être configuré pour déterminer une qualité de la décision du décodeur et adapter de façon incrémentale le paramètre de contrôle de précision des caractéristiques extraites calculées à la qualité déterminée.

La qualité de la décision peut être estimée par exemple par un seuillage sur la probabilité de la décision. Si la décision présente un haut niveau de confiance - elle peut donc être considérée comme sur-satisfaisante - le module de contrôle peut être configuré pour diminuer de façon incrémentale le paramètre de contrôle de précision d'extraction des caractéristiques. Inversement, Si la décision présente un faible niveau de confiance - elle peut donc être considérée comme douteuse - le module de contrôle peut être configuré pour augmenter de façon incrémentale le paramètre de contrôle de précision d'extraction des caractéristiques.

Selon une possibilité, le module de contrôle est configuré pour déterminer le paramètre de contrôle de précision de l'extraction en prenant en compte :
- un ensemble de configurations possibles ordonnées du paramètre de contrôle de précision d'extraction ;
- une valeur courante du paramètre de contrôle de précision d'extraction choisie parmi l'ensemble de configurations possibles du paramètre de contrôle de précision ;
- un incrément dépendant du paramètre de contrôle de qualité définissant le passage de la valeur courante du paramètre de contrôle de précision à une nouvelle valeur du paramètre de contrôle de précision parmi l'ensemble de configurations possibles ordonnées du paramètre de contrôle de précision.

Selon un mode de réalisation, le module de contrôle est configuré pour déterminer le paramètre de contrôle de précision de l'extraction en prenant en compte :
- une valeur courante du paramètre de contrôle de précision d'extraction ;
- une modification du paramètre de contrôle de précision d'extraction, pour un sous-ensemble de caractéristiques extraites aléatoires ou prédéfinies ou basées sur des heuristiques apprises ou prédéfinies dépendant du paramètre de contrôle de qualité.

La présente invention a également pour objet un procédé d'extraction de caractéristiques d'un signal de mesure d'au moins un paramètre physiologique d'un être vivant comprenant :
- Une étape d'acquisition du signal de mesure de l'au moins un paramètre physiologique d'un être vivant ;
- Une étape d'extraction de caractéristiques du signal de mesure ;
- Une étape de décodage utilisant les caractéristiques extraites afin de déterminer un signal de commande d'un actionneur avec lequel l'être vivant interagit ;
- Une étape de pilotage de l'actionneur en fonction du signal de commande dans lequel l'étape d'extraction de caractéristiques du signal de mesure est réalisée de façon approximée en prenant en compte un paramètre de contrôle de précision, le procédé comprenant en outre :
- Une étape de détermination d'un paramètre d'évaluation de qualité ;
- Une étape d'ajustement du paramètre de contrôle de précision en fonction d'une valeur du paramètre d'évaluation de qualité en choisissant ledit paramètre de contrôle de précision correspondant au niveau de précision le plus faible permettant de faire tendre la valeur du paramètre d'évaluation de qualité vers une gamme de valeurs de qualité prédéfinie ou de maintenir la valeur du paramètre d'évaluation de qualité dans une gamme de valeurs de qualité prédéfinie.

### Description brève des figures

L'invention sera mieux comprise à l'aide de la description détaillée qui est exposée ci-dessous en regard du dessin annexé dans lequel :
La [Fig. 1] représente un premier mode de réalisation d'un dispositif électronique selon l'invention ;
La [Fig. 2] représente un deuxième mode ou un troisième mode de réalisation d'un dispositif électronique selon l'invention ;
La [Fig. 3] représente un quatrième mode de réalisation d'un dispositif électronique selon l'invention ;
La [Fig.4] représente un cinquième mode de réalisation d'un dispositif électronique selon l'invention ;
La [Fig.5] représente un sixième mode de réalisation d'un dispositif électronique selon l'invention ;
La [Fig. 6] Illustre la technique d'approximation d'une ondelette avec une approximation linéaire continue par morceaux et son utilisation pour une approximation d'une convolution d'un signal par une ondelette ;
La [Fig. 7] montre une comparaison de deux approximations d'une ondelette.
La [Fig. 8] représente la corrélation de caractéristiques extraites approximées par rapport aux caractéristiques exactes.
La [Fig. 9] représente l'évolution de la précision obtenue en fonction de l'énergie consommée pour différentes valeurs du paramètre de contrôle de précision pour un décodage à 5 classes concernant une sélection d'un doigt de la main.
La [Fig. 10] représente l'évolution de la précision obtenue en fonction de l'énergie consommée pour différentes valeurs du paramètre de contrôle de précision pour un décodage à 2 classes concernant une détermination d'un état de repos ou d'activité.
La [Fig. 11] montre l'influence de la qualité du signal sur la corrélation entre une transformée en ondelette continue (CWT) et une transformée en ondelette continue linéaire par morceaux (PLCWT).
La [Fig. 12] illustre la valeur réellement décodée pour 5 caractéristiques et leurs plages de valeur attendue, dans un état donné.
La [Fig. 13] représente un organigramme d'un procédé de d'extraction de caractéristiques selon l'invention.

### Description détaillée de l'invention

Dans la description détaillée qui va suivre des figures définies ci-dessus, les mêmes éléments ou les éléments remplissant des fonctions identiques pourront conserver les mêmes références de manière à simplifier la compréhension de l'invention.

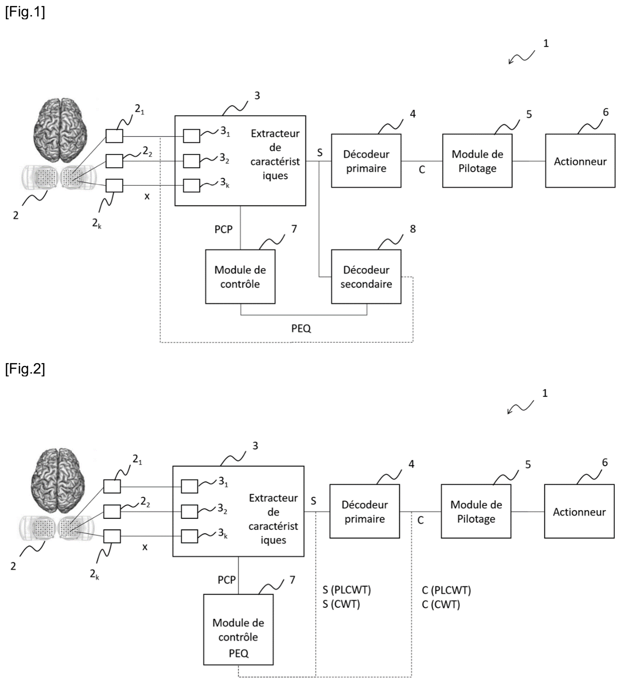

Les figures 1 à 5 représentent différents modes de réalisation d'un dispositif électronique 1 comprenant un ensemble de capteurs primaires 2 configurés pour réaliser l'acquisition d'un premier ensemble de signaux de mesure x d'au moins un paramètre physiologique d'un être vivant. En particulier, selon les modes de réalisation présentés sur les figures, l'ensemble de capteurs primaires 2 peut être un ensemble d'électrodes d'électroencéphalographie (EEG), d'électrocorticographie (ECoG) ou un réseau de microélectrodes (MEA). Nous considérerons par la suite un exemple d'être vivant qui est un utilisateur humain désigné par l'utilisateur.

Le premier ensemble de signaux de mesure x est un ensemble de signaux électrophysiologiques, en particulier des signaux électrophysiologiques émis par le cortex ou signaux neuronaux ou encore réseaux cérébraux.

Les différents types de capteurs présentent des résolutions spatiales variables en fonction du degré d'invasivité. Bien que les électrodes ECoG et EEG capturent toutes deux des signaux qui sont moyennés sur une population de neurones, les signaux ECoG ont une meilleure résolution spatiale. Les réseaux MEA et ECoG sont tous deux implantés par voie chirurgicale, mais l'intervention est moins invasive dans le second cas, car le réseau est placé à la surface du cerveau.

Des études antérieures ont montré que les signaux ECoG ont des modulations de fréquence qui sont couplées à des tâches mentales spécifiques telles que l'intention motrice.

En outre, les signaux cérébraux sont non stationnaires, ce qui signifie que des informations utiles peuvent également être extraites dans le domaine temporel.

Le dispositif électronique 1 comprend un extracteur de caractéristiques 3 configuré pour extraire un deuxième ensemble de caractéristiques S à partir du premier ensemble de signaux de mesure x. L'extracteur de caractéristique (3) est configuré pour pouvoir réaliser l'extraction de caractéristiques (S) du signal de mesure (x) de façon approximée ou en pleine précision selon plusieurs niveaux de précision, le niveau de précision requis étant fonction d'un paramètre de contrôle de précision (PCP) souhaité, la consommation électrique de l'extracteur de caractéristique augmentant avec le niveau de précision ;

Selon une possibilité, une transformée en ondelettes (WT) peut être utilisée pour réaliser cette extraction car elle permet d'extraire des caractéristiques contenant des informations dans le domaine temps-fréquence ou en d'autres termes une information spectro-temporelle.

Le calcul d'une transformée en ondelettes, plus généralement connue sous le nom de transformée en ondelettes continue (CWT), nécessite une convolution entre le signal analysé x et une ondelette *ψₐ* ou son conjugué complexe ${ψ}_{a}^{∗}$.

Cette approche est couteuse en calculs et entraîne une forte consommation d'énergie étant donné que le décodage nécessite l'extraction de ces caractéristiques sur plusieurs électrodes, par exemple entre 16 et 256, et plusieurs bandes de fréquences, par exemple entre 1 et 24. Il est donc nécessaire d'effectuer une convolution ou une corrélation par électrode et par bande de fréquence. Ces caractéristiques sont ensuite utilisées par le décodeur pour la prise de décision.

La transformée en ondelettes continue CWT peut s'exprimer comme suit : $S \left(τ\right) = {\int }_{K} x \left(t+τ\right) {ψ}_{a}^{∗} \left(t\right) dt$ où a est le facteur d'échelle pour une fréquence donnée, τ le décalage temporel de convolution et K l'intervalle sur lequel l'ondelette est définie. Pour les signaux numériques, l'intervalle est discrétisé et l'intégrale devient une somme finie. La transformée discrétisée WT est donc exprimé comme un produit scalaire. $S \left(τ\right) = {\sum }_{i = 1}^{N} x \left(i+τ\right) {ψ}_{a}^{∗} \left(i\right)$

Ainsi, le circuit d'extraction 3 est configuré pour réaliser d'extraction de caractéristiques du signal de mesure en utilisant un calcul approximé prenant en compte le paramètre de contrôle de précision PCP afin de réduire le nombre de calculs à réaliser.

En particulier, si le mode d'extraction correspond au calcul de transformées en ondelette, c'est un calcul d'une convolution entre le signal d'entrée et une ondelette, en prenant en compte un paramètre de contrôle de précision, il est possible d'utiliser un mode de calcul approximé par transformée en ondelettes linéaire continue par morceaux (PLCWT).

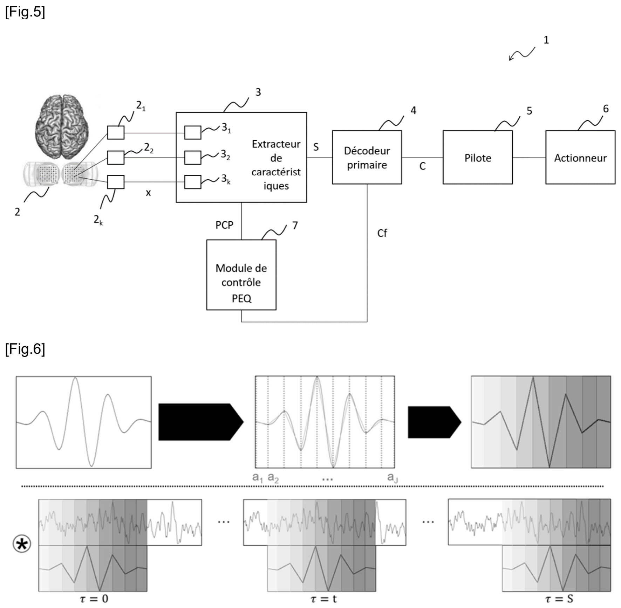

La technique de transformée en ondelettes linéaire continue par morceaux (PLCWT) est basée sur une approximation par morceaux du premier ordre *ϕ* de l'ondelette ${ψ}_{a}^{∗}$ comme le montre la figure 6. L'intervalle d'ondelette est divisé en J - 1 segments et, sur chaque segment i de la subdivision, l'ondelette est approximée par une fonction linéaire *ϕ*ᵢ . L'ensemble des morceaux de fonctions linéaires relie donc J points.

L'application de l'intégration par parties à la définition de la CWT dans l'équation intégrale de la CWT avec l'approximation donne la formule suivante (E4) : $S \left(τ\right) = {X}^{\left(1\right)} \left({a}_{J}+τ\right) {ϕ}_{J - 1} \left({a}_{J}\right) - {X}^{\left(1\right)} \left({a}_{1}+τ\right) {ϕ}_{1} \left({a}_{1}\right) - {\int }_{{a}_{1}}^{{a}_{J}} {X}^{\left(1\right)} \left(t+τ\right) ϕ ′ \left(t\right) dt$

Où *X*⁽¹⁾ est la première primitive du signal et *ϕ'* la dérivée de l'ondelette approximée par morceaux *ϕ*. Le dernier terme intégral, noté *I*(*τ*), est alors calculé sur les segments de subdivision sur lesquels l'approximation de l'ondelette est linéaire par morceaux. La dérivée *ϕ'ᵢ* sur chaque segment est constante et peut être retirée de l'intégrale comme indiqué dans (E4'). $I \left(τ\right) = {\sum }_{i = 1}^{J - 1} ϕ {′}_{i} {\int }_{{a}_{i}}^{{a}_{i + 1}} {X}^{\left(1\right)} \left(t+τ\right) dt$ $I \left(τ\right) = {\sum }_{i = 1}^{J - 1} ϕ {′}_{i} \left[{X}^{\left(2\right)}\left({a}_{i + 1}+τ\right)-{X}^{\left(2\right)}\left({a}_{i}+τ\right)\right]$ $I \left(τ\right) = {\sum }_{i = 1}^{J} {b}_{i} {X}^{\left(2\right)} \left({a}_{i}+τ\right)$

*X*⁽²⁾ est la seconde anti-dérivée du signal et les coefficients réordonnés *bᵢ* de la somme sont donnés comme suit : ${b}_{i} = \left\{\begin{matrix}{b}_{1} = - ϕ {′}_{1} \\ ϕ {′}_{i - 1} - ϕ {′}_{i} pour i ∈ \left‖2,J-1\right‖ \\ {b}_{J} = ϕ {′}_{J - 1}\end{matrix}\right.$

En pratique, pour calculer cette convolution, le signal est intégré deux fois à l'aide d'une intégration trapézoïdale ou rectangulaire présentant un coût de calcul négligeable devant les autres traitements. En nommant L la longueur du signal, et en rappelant que J est le nombre de points utilisés pour approximer l'ondelette, le calcul nécessite L×J additions et multiplications pour calculer la somme en (E4'). Des optimisations supplémentaires sont possibles en fonction du type d'ondelette choisi.

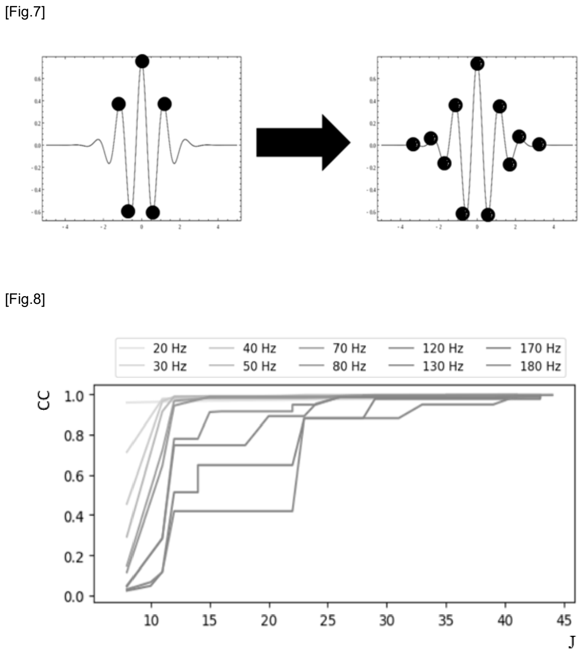

Sur la Figure 7, deux approximations possibles d'une ondelette avec cette technique sont représentées. Sur le schéma de gauche de la figure 7, la fonction est discrétisée en utilisant très peu de points. Les variations importantes de l'ondelette sont centrées dans un voisinage de zéro. Les points d'approximation seront donc choisis principalement dans cette zone. La distribution peut être élargie, en ajoutant des points vers les extrémités, comme cela est figurée sur le schéma de droite de la figure 7. Cette discrétisation est utilisée pour calculer la convolution avec le signal d'entrée x. En diminuant le nombre de points de subdivision, on réduit le nombre de calculs nécessaire. Si on utilise l'approximation de l'ondelette sur le schéma de gauche de la figure 7, avec peu de points, la corrélation avec le résultat théorique (CWT) utilisant l'ondelette non approximée sera moins élevée que la version à droite avec plus de points.

Le choix du nombre de points utilisés pour approximer une ondelette est un exemple de paramètre de contrôle de précision PCP qui permet d'influencer la qualité des caractéristiques extraites S.

L'extracteur de caractéristiques 3 est ainsi configuré pour réaliser d'extraction de caractéristiques S du signal de mesure x en prenant en compte un paramètre de contrôle de précision PCP. Dans le cas de la technique PCWT, le paramètre de contrôle de précision correspond au nombre de points de subdivision fixé pour approximer l'ondelette par morceaux.

Pour le décodage BCI, il est nécessaire d'extraire de l'information à des fréquences différentes. Selon une possibilité, l'extracteur peut comprendre un seul module d'extraction de caractéristiques qui extrait les caractéristiques en série, une à une. Cela pourrait être le cas si un microcontrôleur était utilisé pour l'extraction. Selon une autre possibilité, le circuit d'extraction des caractéristiques 3 comprend une pluralité de modules d'extraction 3k configurés pour extraire des caractéristique S. Il peut également comprendre des modules distincts pour traiter les signaux d'entrée x provenant d'électrodes distinctes 2k. Selon différents modes de réalisation selon la seconde possibilité, le nombre d'extracteurs de caractéristiques peut être compris entre N si l'on extrait N types de caractéristiques différentes, et au plus N x K, K étant le nombre d'électrodes. Pour les différentes fréquences extraites on peut utiliser le même module de calcul en changeant les coefficients multiplicatifs du module.

La pluralité de caractéristiques extraites S correspond à une pluralité de fréquences et/ou une pluralité de localisation spatiales, correspondant par exemple au positionnement des électrodes. Plusieurs caractéristiques extraites S peuvent aussi correspondre à des instants spécifiques τ.

Ainsi, des ondelettes de fréquences différentes sont utilisées. Une ondelette de haute fréquence aura un nombre plus important de sommets par intervalle de temps qu'une ondelette de basse fréquence. Pour approximer les ondelettes de haute fréquence il faudra donc plus de points que pour celles de basse fréquence, afin d'obtenir des approximations de qualité comparable.

Un exemple de façon d'évaluer la qualité des caractéristiques extraites approximées S est par le niveau de corrélation avec les caractéristiques exactes. En particulier, Cette corrélation peut être obtenue en réalisant un calcul exact de façon périodique, par exemple toutes les M itérations (par exemple 1000) ce qui permet de faire des vérifications régulières. La mesure de corrélation peut être faite séparément pour chaque bande de fréquence et pour chaque électrode. En agissant sur le niveau de corrélation pour chaque fréquence, cela permettrait une adaptation fine et précise de la qualité des caractéristiques extraites, en fonction des exigences de la tâche et en fonction de la variabilité des signaux entre différents patients ou pour un même patient au cours du temps. Par exemple, pour des fréquences qui contiennent peu d'information mais qui sont tout de même utiles ou nécessaires pour la tâche, le niveau de corrélation peut être réduit, ce qui permet de réaliser des économies d'énergie supplémentaires tout en conservant l'information utile extraite. Pour des fréquences plus importantes pour la tâche, le niveau de corrélation peut être augmenté, assurant ainsi une performance optimale.

La Figure 8 montre la corrélation des caractéristiques calculées par rapport aux caractéristiques exactes. Chaque ligne correspond à une fréquence d'ondelette différente. Les abscisses représentent le nombre de points J de subdivision utilisés pour approximer l'ondelette selon la technique PLCWT. Les ordonnées représentent le coefficient de corrélation CC. Sur la droite de la figure, on peut observer qu'une utilisation de 45 points de subdivision permet d'obtenir un coefficient de corrélation de 1, c'est-à-dire la même valeur, à une transformation affine près, qu'avec une utilisation des ondelettes exactes CWT. Sur la gauche de la figure, on peut observer qu'avec seulement 10 points pour approximer, les valeurs du coefficient de corrélation sont plus faibles, donc que l'approximation est plus mauvaise. Au milieu de la figure, avec 20 points de subdivision, on peut observer que les basses fréquences (<70Hz) donnent des niveaux de corrélation plus élevés que les hautes fréquences (>120Hz).

Différentes autres techniques peuvent être utilisées en remplacement de la technique PLCWT décrite ci-dessus pour extraire des caractéristiques S de façon approximée en adaptant la qualité du résultat à un paramètre de contrôle de précision PCP.

Ainsi, selon une première possibilité, une transformée en cosinus discrète peut être utilisée. Dans ce cas, le paramètre de contrôle de précision PCP peut correspondre à un nombre de points utilisés par la transformée en cosinus discrète ou à la précision numérique, c'est à dire le nombre de bits utilisés pour le codage des informations.

Selon une autre possibilité, une transformée en ondelette continue mais avec un niveau de quantification adaptatif de type QCWT (encore appelée CWT quantifiée) peut être utilisée. Dans ce cas, le paramètre de contrôle de précision PCP peut correspondre à la précision numérique de l'approximation de l'ondelette.

Selon une autre possibilité, une transformée de Fourier rapide avec résolution adaptative peut être utilisée, la résolution pouvant être utilisée comme paramètre de contrôle de précision PCP. En effet, quand une réduction du nombre de points dans un transformée de Fourier rapide réduit la résolution fréquentielle mais également le nombre d'opérations arithmétiques et donc l'énergie de calcul.

Selon une autre possibilité, des filtres numériques avec des supports de taille variable peuvent être utilisés. La qualité d'un filtre numérique, par exemple un filtre du type à réponse impulsionnelle finie ou infinie dépend du nombre de coefficients du filtre. Ainsi, l'extracteur peut disposer de plusieurs filtres pré-calculés avec des nombres de coefficients différents. Le paramètre de contrôle de précision PCP permet de choisir un filtre parmi la pluralité de filtres en fonction de la qualité désirée.

Selon une autre possibilité, un ajustement de la précision numérique, notamment du nombre de bits, peut être utilisée dans les calculs. Quand on réduit le nombre de bits utilisés pour représenter un signal, on introduit un bruit de quantification. Le nombre de bits de précision peut être modifié en fonction d'un paramètre de contrôle de précision PCP à la volée, en fonction des besoins.

Selon une autre possibilité, une arithmétique approximative ou d'autres approches de calcul approximatifs peuvent être utilisés en adaptant le niveau d'approximation à une valeur du paramètre de contrôle de précision PCP. Des méthodes de calcul de calcul approximatif sont décrites par exemple dans le document M. Shafique et al., DAC '16: Proceedings of the 53rd Annual Design Automation Conference, Article No.: 99, Pages 1 - 6 (ex. https://dl.acm.org/doi/10.1145/2897937.2906199).

Le dispositif électronique 1 comprend un décodeur primaire 3 ou circuit de décodage.

Le décodeur primaire 3 est configuré pour utiliser les caractéristiques extraites S produites par l'extracteur de caractéristique 3 pour déterminer un signal de commande C. Le décodeur primaire 3 peut notamment utiliser un modèle de décodage comme par exemple les machines à vecteurs de support, les forêts d'arbre de décision ou les réseaux neuronaux artificiels.

La plupart des modèles de décodage pouvant être utilisés par le circuit de décodage sont insensibles aux transformations affines des entrées. Donc, un niveau de corrélation linéaire élevé entre les caractéristiques approximées et théoriques est suffisant pour assurer que le décodeur aurait de bonnes performances. Cependant, il peut exister des cas où la précision de décodage est élevée malgré le fait que les caractéristiques utilisées sont faiblement corrélées aux caractéristiques théoriques.

En effet, le niveau de corrélation d'une approximation est un indicateur de la qualité de l'information obtenue. Cependant, une métrique plus fiable de l'évaluation du système est la précision de décodage.

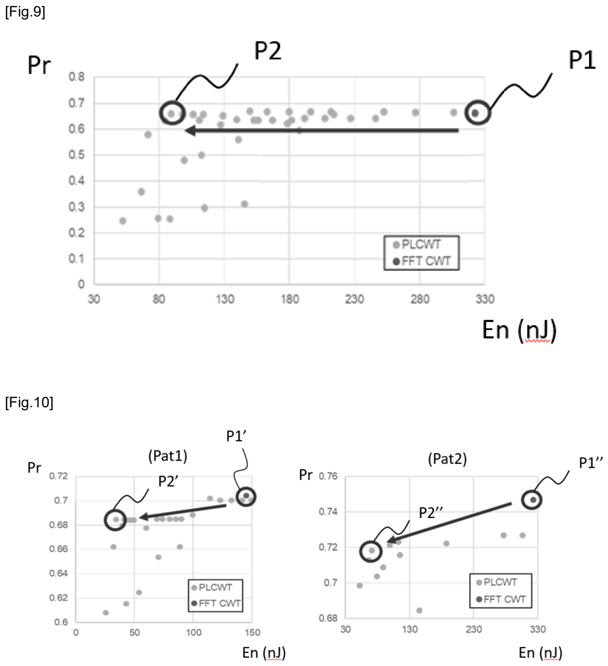

Par exemple, pour une tâche de décodage à 5 classes correspondant à une sélection de doigts par l'utilisateur, la précision obtenue Pr avec plusieurs choix de paramètres de qualité, et en particulier de subdivisions J pour approximer les ondelettes peut être calculée, en lien avec l'énergie nécessaire Eₙ en nJ pour évaluer la convolution pour chaque subdivision choisie. La figure 9 reprend le résultat de ces calculs selon un exemple de réalisation. Nous constatons qu'il existe des approximations qui permettent de maintenir la même précision (à 1% près), tout en consommant 3,5X moins d'énergie en comparant le point P1 du graphique qui correspond à une transformée de type CWT réalisée par transformée de Fourier rapide et le point P2 qui utilise une approximation par PLCWT.

Selon un autre exemple illustré par la figure 10, pour une tâche de décodage à 2 classes correspondant à une détermination d'un état de repos ou d'activité, l'énergie consommée peut être réduite de 4X avec une perte de précision de 2% pour un premier utilisateur Pat1 en comparant le point P1' du graphique qui correspond à une transformée de type CWT réalisée par transformée de Fourier rapide et le point P2' qui utilise une approximation par PLCWT, et de 5X avec une perte de précision de seulement 3% pour un deuxième utilisateur Pat2 en comparant le point P1" du graphique qui correspond à une transformée de type CWT réalisée par FFT et le point P2" qui utilise une approximation par PLCWT. Il est important de noter qu'il existe de nombreuses approximations intermédiaires, qui permettent de fixer un compromis acceptable entre le gain énergétique et la perte de précision.

En résumé, on est capable de trouver des valeurs d'approximation des ondelettes qui permettent de réduire la complexité calculatoire en gardant une précision de décodage élevé. Nous rappelons que la technique PLCWT n'est qu'un exemple d'une caractéristique dont on peut moduler la qualité.

Le dispositif électronique 1 comprend également un actionneur 5 avec lequel l'être vivant interagit, qui peut être notamment un stimulateur de moelle épinière, un stimulateur des muscles de l'utilisateur comme par exemple un stimulateur utilisé dans les traitements de stimulation électrique fonctionnelle - ou Functional Electrical Stimulation / FES en anglais, un actionneur d'exosquelette, un générateur de parole, un actionneur de fauteuil roulant, tel qu'un moteur de fauteuil roulant, un afficheur configuré pour afficher des informations décodées à partir de signaux neuronaux de l'utilisateur. Le dispositif comprend également un circuit de pilotage de l'actionneur configuré pour piloter l'actionneur en fonction du signal de commande C du décodeur primaire 4.

Le dispositif comprend en outre un module de contrôle 7 configuré pour mesurer ou collecter un paramètre d'évaluation de qualité (PEQ) de l'action de l'actionneur en interaction avec ledit être vivant, le module de contrôle étant en outre configuré pour ajuster le paramètre de contrôle de précision PCP en fonction d'une valeur du paramètre d'évaluation de qualité PEQ.

Le module de contrôle 7 est configuré pour ajuster le paramètre de contrôle de précision PPPCP en fonction d'une valeur du paramètre d'évaluation de qualité PEQ en choisissant ledit paramètre de contrôle de précision PCP correspondant au niveau de précision le plus faible permettant de faire tendre la valeur du paramètre d'évaluation de qualité vers une gamme de valeurs de qualité prédéfinie ou de maintenir la valeur du paramètre d'évaluation de qualité dans une gamme de valeurs de qualité prédéfinie.

Le paramètre d'évaluation de qualité PEQ et la gamme de valeurs de qualité peuvent être déterminé de plusieurs façon selon plusieurs modes de réalisations décrits ci-dessous.

Selon un premier mode de réalisation tel que représenté sur la figure 1, le dispositif électronique 1 comprend un décodeur secondaire 8 de niveau de satisfaction d'un utilisateur configuré pour fournir un signal de satisfaction de l'utilisateur, le paramètre d'évaluation de qualité PEQ prenant en compte ce signal de satisfaction de l'utilisateur (ou étant par exemple égal à ce signal de satisfaction). Dans ce cas, la gamme de valeurs de qualité prédéfinie peut correspondre à des valeurs au-dessus d'une valeur de satisfaction minimale de l'utilisateur (ou à une simple valeur positive de satisfaction si le signal de satisfaction est binaire). A titre d'exemple, le décodeur secondaire utilisé peut être du type décrit dans la demande de brevet déposée sous le numéro FR2415312. Selon une possibilité, le décodeur secondaire 8 de niveau de satisfaction d'un utilisateur est configuré pour réaliser un décodage de caractéristiques extraites S de signaux électrophysiologiques émis par le cortex (ou signaux neuronaux) fournies par l'extracteur de caractéristiques 3. Le décodage peut être réalisé en temps réel.

Selon une autre possibilité, le décodeur secondaire est configuré pour réaliser un décodage sur la base des signaux d'entrée x, ou de caractéristiques simples à extraire sur la base de ces signaux, par exemple la moyenne et/ou la variance de ces signaux, qui peuvent être suffisantes pour décoder un niveau de satisfaction. Ainsi, les signaux d'entrée x peuvent être traités directement par le décodeur secondaire ou par un module complémentaire simple.

Selon une autre possibilité, il serait possible de configurer le décodeur secondaire pour prendre en compte à la fois les caractéristiques S extraites par l'extracteur de caractéristiques 3 et les caractéristiques simples, par exemple de type moyenne ou variance, obtenues directement à partir du signal d'entrée x.

Selon une possibilité, l'état de satisfaction de l'utilisateur est décodé quand il exécute une tâche. Si, lors du décodage, le décodeur secondaire identifie que l'utilisateur n'est pas satisfait, le module de contrôle 7 est configuré pour modifier le paramètre de contrôle de précision PCP afin d'incrémenter progressivement la précision des calculs et d'améliorer la valeur du paramètre d'évaluation de qualité PEQ, c'est-à-dire la satisfaction de l'utilisateur. Ainsi, ce signal peut être utilisé pour contrôler dynamiquement la qualité des caractéristiques.

Le décodeur secondaire 8 peut être configuré pour fonctionner avec une fréquence plus basse que le décodeur principal et/ou en effectuant moins de calcul que le décodeur principal.

Le critère utilisé pour déterminer les puissances et fréquence de calcul utilisables par le décodeur secondaire 8 peut être défini en comparant :
- la puissance PP consommée par la chaine principale de traitement configurée pour déterminer la commande C comprenant les capteurs, l'extracteur de caractéristiques 3, le décodeur primaire 4 avec le paramètre de contrôle de précision PCP courant ;
- la puissance PPF consommée par les traitements effectués par la chaine principale de traitement configurée pour déterminer la commande C comprenant les capteurs, l'extracteur de caractéristiques 3, le décodeur primaire 4 en pleine précision ;
- l'énergie ES consommée par la chaine de retour comprenant le décodeur secondaire, le module de contrôle 7 et/ou les autres éléments visant à déterminer le Paramètre d'évaluation de qualité PEQ et à modifier le paramètre de contrôle de précision PCP.
- La fréquence FS à laquelle est effectué les traitements de la chaine de retour. Ainsi, le critère peut être défini de la façon suivante : $ES \times FS + PP < PPF$

Cette relation ou critère énergétique E5 traduit le fait que l'énergie ou la puissance consommée par la chaine principale et la chaine secondaire en utilisant une approximation correspondant à une valeur courante du paramètre de contrôle de précision PCP est inférieure, et de préférence très inférieure, à l'énergie ou la puissance consommée par la chaine principale en pleine précision. Cette relation est applicable au premier mode de réalisation présenté ci-dessus, mais également à l'ensemble des modes de réalisation présentés ci-après.

Selon un deuxième mode de réalisation représenté sur la figure 2, le module de contrôle 7 est configuré pour réaliser périodiquement un calcul d'extraction de caractéristique S exact ou pour commander un extracteur de caractéristiques 3 afin de réaliser périodiquement un calcul d'extraction de caractéristique S exact. Le module de contrôle est configuré pour, suite au calcul exact, mesurer l'écart entre le résultat obtenu avec une extraction de caractéristiques exactes et celui obtenu avec une extraction de caractéristiques S approximées avec le niveau du paramètre de contrôle de précision d'extraction PCP courant de façon à déterminer une valeur du paramètre d'évaluation de qualité PEQ. Selon une possibilité, l'écart peut être évalué par le calcul d'une corrélation CC des caractéristiques extraites exactes et approximées.

Selon une autre possibilité, l'écart peut être évalué en comparant le résultat du décodage réalisé par le décodeur primaire, par exemple en identifiant si le résultat du décodage est le même avec les caractéristiques extraites approximées et des caractéristiques extraites exactes, notamment sur la détermination d'une classe en sortie.

Le calcul exact est réalisé par exemple, pour un epoch sur 1000.

Dans ce mode de réalisation, la gamme de valeurs de qualité prédéfinie peut correspondre à une valeur maximale de l'écart autorisé.

Le paramètre de contrôle de précision PCP est calculé comme fonction continue ou discrète du paramètre d'évaluation de qualité PEQ. La figure 8 montre l'influence de la qualité du signal sur le coefficient de corrélation (CC) entre la CWT et la PLCWT, pour une transformée utilisant une ondelette (théorique et approximée) de 60 Hz.

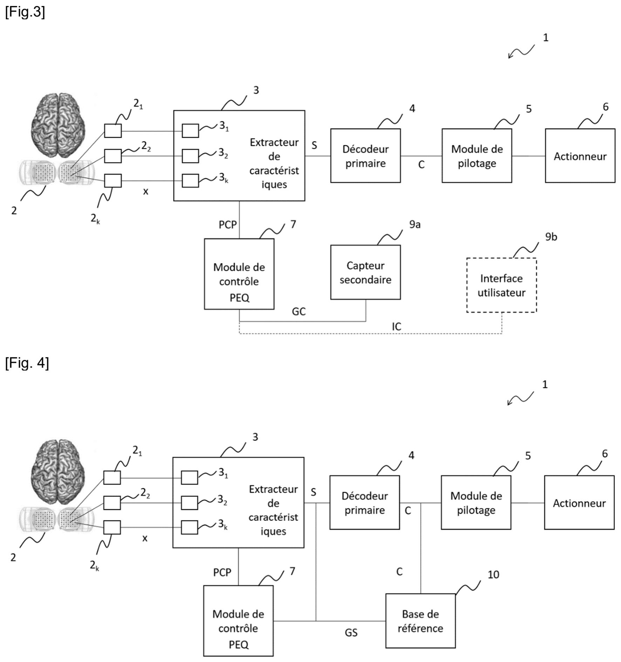

Selon un troisième mode de réalisation représenté sur la figure 3, le module de contrôle 7 est configuré pour déterminer le paramètre d'évaluation de qualité PEQ en prenant en compte une valeur d'un indicateur de performance correspondant à une « vérité terrain ». Selon différents exemples, un indicateur de performance peut être un taux de réussite des décodages, une précision des actions effectuées ou la variabilité des valeurs obtenues lors du décodage. Selon une possibilité, si la performance diminue, le module de contrôle 7 peut augmenter la qualité des caractéristiques extraites pour améliorer la précision. Comme cela est représenté sur la figure 4, le dispositif électronique peut comprendre un capteur de mesure secondaire 9a configuré pour acquérir une grandeur de contrôle GC, le module de contrôle étant configuré pour déterminer le paramètre d'évaluation de qualité en prenant en compte un indicateur de performance déterminé à partir d'une valeur de la grandeur de contrôle. Selon une autre possibilité représentée en pointillé sur la même figure, le dispositif électronique peut comprendre une interface utilisateur 9b permettant de collecter une information de contrôle IC provenant d'un utilisateur.

Selon un premier exemple, le capteur de mesure secondaire 9a peut être un capteur de position, ou une pluralité de capteurs de position configuré(s) pour identifier des positions anormales d'éléments contrôlés par l'actionneur.

Selon un second exemple, le capteur de mesure secondaire 9a peut être un capteur de mouvement, ou une pluralité de capteurs de mouvement configuré(s) pour analyser le mouvement des effecteurs contrôlé par les actionneurs, comme l'exosquelette, ou les membres de l'utilisateur et discriminer des écarts à des scénarios connus tels que la marche, la course, ou la saisie d'objet.

Selon un troisième exemple, l'interface utilisateur 9b peut être un capteur d'interface utilisateur, comme par exemple un rhéostat similaire à un bouton de volume, qui permet à l'utilisateur de donner une valeur qui correspond au paramètre d'évaluation de qualité en fonction de son ressenti.

Dans ce cas, la gamme de valeurs de qualité prédéfinie peut correspondre à une valeur maximale de l'écart autorisé entre l'indicateur de performance et une valeur nominale ou encore à une zone supérieure à une valeur de seuil. A titre d'exemple, si un mouvement est commandé par l'actionneur et qu'une mesure de ce mouvement est réalisée, le paramètre d'évaluation de qualité peut-être un angle mesuré, et la gamme de valeur de qualité une gamme ou plage angulaire prédéfinie à atteindre.

A titre d'exemple, pour une extraction de caractéristiques utilisant la méthode PLCWT, en considérant que le paramètre de contrôle de précision correspond au nombre de points d'approximation, à valeurs entre 5 et 15 points d'approximation. Le paramètre d'évaluation de qualité peut être défini comme précédemment comme l'écart entre l'angle prédéfini et l'angle mesuré, en degrés : $PEQ = \left|angle prédéfini-angle mesuré\right|$

La détermination du paramètre de contrôle de précision est réalisée par exemple de la façon suivante :
Si PEQ <= 10, PCP = PCP - 1
Si PEQ > 10, PCP = min(PCP + (PEQ-10), 15 )

Dans cet exemple, la gamme de valeurs de qualité peut être définie comme PEQ<=10.

Le PCP est proportionnel au PEQ, mais, selon d'autres applications, une fonction plus complexe avec un passage à l'échelle ou des offsets peuvent être nécessaires afin d'établir la correspondance entre le PCP et le PEQ.

Selon un quatrième mode de réalisation représenté sur la figure 4, le module de contrôle 7 est configuré pour déterminer le paramètre d'évaluation de qualité en prenant en compte une détection d'anomalie. A titre d'exemple, cette détection d'anomalie peut se baser sur la comparaison entre les valeurs ou amplitudes des caractéristiques extraites S et une référence RS de valeurs attendues pouvant être extraite d'une base de référence 10 comprise dans le dispositif électronique 1 ou accessible par ce dispositif. Chaque référence RS peut avoir été déterminée par exemple lors de l'apprentissage, de façon à mettre en correspondance un état de sortie du décodeur primaire 4 avec des valeurs attendues pour les caractéristiques extraites. A titre d'exemple, il est possible que les références RS correspondent à des gammes de valeurs. Une telle gamme de valeur RS peut être déduite en considérant la distribution des valeurs de la caractéristique extraite S pendant l'apprentissage, en calibrant par exemple une distribution gaussienne sur la base des valeurs constatées, et en définissant une gamme de valeur comme étant centrée sur la moyenne et présentant une largeur correspondant à un multiple ou à un sous-multiple de l'écart-type de la distribution.

Ainsi, en fonction de la valeur de la commande C ou de l'état déterminé par le décodeur primaire, le module de contrôle peut accéder aux références RS correspondantes dans la base de référence 10 qui permettent de connaître des attendus sur les valeurs des caractéristiques.

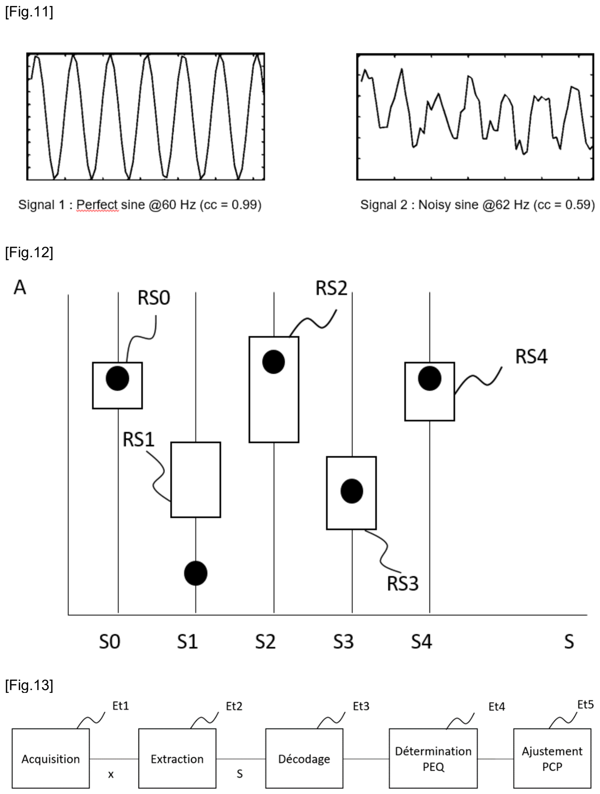

La figure 12 illustre un exemple de réalisation de la détection d'anomalie. Les valeurs des caractéristiques S, en particulier de caractéristiques S0 à S4, sont représentées par des points noirs. En pratique, les valeurs de ces caractéristiques extraites S peuvent correspondre à des valeurs de puissance ou d'énergie spectrale dans différentes bandes de fréquence.

Ainsi, dans l'exemple de la figure 12, le module de contrôle peut comparer la valeur de chaque caractéristique extraite S avec la référence RS correspondante, constituée par exemple par une gamme de valeur attendue relative à l'état ou la commande C considérée. Il apparaît dans cet exemple que la valeur pour S1 (qui peut correspondre à une fréquence donnée, par exemple de 10 Hz) est en dehors de la référence ou gamme attendue RS1 pour l'état ou la valeur de commande considérée. Dans ces conditions, le module de contrôle peut être configuré pour déterminer le paramètre d'évaluation de qualité PEQ, par exemple comme un vecteur comprenant des indications sur les anomalies potentiellement détectées sur chaque composante ou sur le degré de correspondance avec la référence RS, et pour modifier le paramètre de contrôle de précision PCP en conséquence. En particulier, dans l'exemple de la figure 12, le module de contrôle pourrait être configuré pour modifier le paramètre de contrôle de précision spécifiquement pour la caractéristique S1 afin d'augmenter le niveau de précision de calcul de cette caractéristique. En cas d'absence d'anomalie ou si les valeurs constatées présentent un degré important de conformité avec la référence, il est possible de diminuer le paramètre de contrôle de précision pour tester un calcul moins énergivore.

La détection des anomalies peut être réalisée par bande de fréquence ou par caractéristique extraite. Ainsi, le module de contrôle est configuré pour identifier les fréquences ou les caractéristiques, impliquées dans une tâche donnée, qui nécessitent une amélioration. Selon une possibilité, le module de contrôle peut améliorer la qualité des caractéristiques extraites pour les fréquences spécifiques identifiées.

Dans ce mode de réalisation, la gamme de valeurs de qualité prédéfinie peut correspondre à une valeur maximale de l'écart autorisé entre les valeurs du paramètre d'évaluation de qualité et les références RS.

Selon un cinquième mode de réalisation représenté sur la figure 5, le module de contrôle est configuré pour déterminer le paramètre d'évaluation de qualité en prenant en compte une valeur d'un niveau de confiance Cf ou de probabilité d'un résultat du décodeur primaire. Ces dispositions permettent d'utiliser les probabilités fournies par le décodeur primaire comme niveau de confiance en la décision émise. Par exemple, des décodeurs comme des classificateurs linéaires ou des arbres de décision associent une probabilité à une classe majoritaire choisie. Cette probabilité pourrait être utilisée pour contrôler le niveau de qualité des caractéristiques extraites. Selon une possibilité, le module de contrôle peut être configuré pour associer le niveau de confiance ou probabilité d'une classe décodée à un niveau de qualité nécessaire au décodage suivant, et ainsi, déterminer à chaque étape le paramètre de contrôle de précision PCP. Selon une autre possibilité, le module de contrôle peut être configuré pour déterminer une qualité de la décision du décodeur et adapter de façon incrémentale le paramètre de contrôle de précision. La qualité de la décision peut être estimée par exemple par un seuillage sur la probabilité de la décision. Si la décision présente un haut niveau de confiance - elle peut donc être considérée comme sur-satisfaisante - le module de contrôle peut être configuré pour diminuer de façon incrémentale le paramètre de contrôle de précision d'extraction des caractéristiques. Inversement, si la décision présente un faible niveau de confiance - elle peut donc être considérée comme douteuse - le module de contrôle peut être configuré pour augmenter de façon incrémentale le paramètre de contrôle de précision d'extraction des caractéristiques.

Dans ce mode de réalisation, la gamme de valeurs de qualité prédéfinie peut correspondre à une valeur maximale de l'écart autorisé entre les valeurs du paramètre d'évaluation de qualité et les références RS pour un état ou une valeur de commande donnée.

Selon certains modes de réalisation décrits ci-dessus, il est possible que le paramètre d'évaluation de qualité PEQ corresponde à une seule valeur pour l'ensemble des caractéristiques extraites S et/ou pour l'ensemble des paramètres de contrôle de précision PCP.

Cette valeur peut être binaire ne prenant que deux valeurs (retour positif ou négatif). Dans ce cas, la gamme de valeurs de qualité prédéfinie correspond à la seul valeur positive.

Cette valeur peut également être une valeur discrète ou bien une valeur continue. Dans ce cas, la gamme de valeur de qualité peut être par exemple définie comme une zone supérieure à un seuil.

Ainsi, selon une première possibilité, le module de contrôle peut être configuré pour déterminer le paramètre de contrôle de précision PCP de l'extraction en prenant en compte un ensemble de configurations possibles ordonnées du paramètre de contrôle de précision d'extraction PCP. A titre d'exemple, dans le cas d'une extraction de caractéristiques du type transformée en ondelette approximée de type PLCWT sur plusieurs bandes de fréquence, on peut prévoir de définir des valeurs du paramètre de contrôle de précision PCP pour les différentes fréquences selon plusieurs configurations. Il est possible par exemple de prévoir les valeurs de subdivisions ou de nombres de points suivantes pour plusieurs valeurs de fréquence. Pour expliquer ce principe, nous pouvons isoler deux valeurs de fréquence à 10 Hz et 180 Hz, les configurations pouvant être :
- Configuration 1 : 10 Hz = 5 points ; 180 Hz = 25 points
- Configuration 2 : 10 Hz = 10 points ; 180 Hz = 35 points
- Configuration 3 : 10 Hz = 15 points ; 180 Hz = 45 points
- Configuration N : calcul de la CWT exacte

En prenant en compte la valeur courante du paramètre de contrôle de précision d'extraction PCP qui correspond à une configuration parmi l'ensemble de configurations possibles du paramètre de contrôle de précision d'extraction, il est possible de changer la configuration en incrémentant pour passer d'une configuration à la suivante dans un sens ou dans l'autre en fonction de la valeur du paramètre de contrôle de qualité PEQ d'extraction qui définit ainsi le passage de la valeur courante du paramètre de contrôle de précision d'extraction à une nouvelle valeur du paramètre de contrôle de précision d'extraction.

Ainsi, en repérant selon le premier mode de réalisation, une insatisfaction de l'utilisation en tant que retour ou paramètre de contrôle de qualité PEQ, si le paramètre de contrôle de précision PCP d'extraction correspondait à la configuration 2, le module de contrôle peut modifier les paramètres de qualité pour passer à la configuration 3 pour augmenter la qualité de l'extraction des caractéristiques S.

Selon une autre possibilité, le module de contrôle 8 peut être configuré pour modifier le paramètre de contrôle de précision PCP de l'extraction sur la base d'un retour concernant le paramètre de contrôle de qualité PEQ en prenant en compte une valeur courante du paramètre de contrôle de précision d'extraction PCP correspondant par exemple à une configuration donnée de paramètres de subdivisions/nombre de points par fréquence, et une modification du paramètre de contrôle de précision d'extraction, pour un sous-ensemble de caractéristique extraites aléatoire (avec une gestion de priorité round-robin) ou prédéfini ou basé sur des heuristiques apprises ou prédéfinies dépendant du paramètre de contrôle de qualité d'extraction pour augment ou diminuer la qualité de l'extraction.

Ainsi, selon les deux possibilités mentionnées ci-dessus, le paramètre de contrôle de qualité PEQ permettrait de choisir une configuration et donc une puissance de calcul nécessaire pour un bon compromis entre coût et précision de décodage.

Le dispositif électronique 1 peut comprendre un ou plusieurs circuits intégrés qui comprennent l'extracteur de caractéristiques 2, le module de contrôle 3, le décodeur primaire 4, et optionnellement le décodeur secondaire 8.

Les différents modes de réalisation du dispositif électronique décrits précédemment permettent la mise en œuvre d'un procédé d'extraction de caractéristiques d'un signal de mesure x tel que représenté sur la figure 13 comprenant :
- Une étape d'acquisition Et1 d'un signal de mesure ;
- Une étape d'extraction Et2 de caractéristiques du signal de mesure ;
- Une étape de décodage Et3 utilisant les caractéristiques extraites S afin de déterminer un signal de commande C d'un actionneur ;

Dans lequel l'étape d'extraction de caractéristiques du signal de mesure est réalisée de façon approximée en prenant en compte un paramètre de contrôle de précision PCP, le procédé comprenant en outre :
- Une étape de détermination Et4 (par le calcul, la mesure ou de collecte) d'un paramètre d'évaluation de qualité PEQ ;
- Une étape d'ajustement Et5 du paramètre de contrôle de précision (PCP) en fonction d'une valeur du paramètre d'évaluation de qualité.

Le dispositif et le procédé décrit ci-dessus peuvent s'appliquer, comme évoqué précédemment aux Interfaces cerveau-ordinateur (BCIs), afin d'améliorer l'efficacité énergétique des systèmes BCI, permettant des applications moins énergivores et portables.

Le dispositif et le procédé décrit ci-dessus peuvent également s'appliquer au traitement du signal biomédical : La capacité à ajuster dynamiquement la qualité des caractéristiques extraites peut être appliquée à d'autres domaines du traitement du signal biomédical, comme EMG (électromyographie) ou l'électrocardiographie (ECG).

Le dispositif et le procédé décrit ci-dessus peuvent s'appliquer aux systèmes de contrôle de crises épileptiques afin d'analyser les signaux cérébraux en temps réel et détecter les crises épileptiques avec une précision accrue tout en minimisant la consommation d'énergie.

Le dispositif et le procédé décrit ci-dessus peuvent s'appliquer à d'autres domaines ou une extraction de caractéristiques à partir de signaux est réalisée.

## Revendications

1. Dispositif électronique (1) comprenant :
- Un capteur primaire (2) configuré pour réaliser l'acquisition d'un signal de mesure (x) d'au moins un paramètre physiologique d'un être vivant ;
- Un extracteur de caractéristique (3) configuré pour extraire une caractéristique (S) du signal de mesure (x) ;
- Un décodeur primaire (4) utilisant la caractéristique extraite (S) et configuré pour déterminer un signal de commande (C) ;
- Un actionneur (6) avec lequel l'être vivant interagit ;
- Un module de pilotage (5) de l'actionneur (6) configuré pour piloter l'actionneur en fonction du signal de commande (C) déterminé par le décodeur primaire (4) ;
dans lequel l'extracteur de caractéristique (3) est configuré pour pouvoir réaliser l'extraction de caractéristiques (S) du signal de mesure (x) de façon approximée selon plusieurs niveaux de précision, le niveau de précision requis étant fonction d'un paramètre de contrôle de précision (PCP) souhaité, la consommation électrique de l'extracteur de caractéristique augmentant avec le niveau de précision ;
le dispositif comprenant en outre un module de contrôle (7) configuré pour déterminer un paramètre d'évaluation de qualité (PEQ) de l'action de l'actionneur en interaction avec ledit être vivant ;
le module de contrôle (7) étant configuré pour ajuster le paramètre de contrôle de précision (PCP) en fonction d'une valeur du paramètre d'évaluation de qualité (PEQ) en choisissant ledit paramètre de contrôle de précision (PCP) correspondant au niveau de précision le plus faible permettant de faire tendre la valeur du paramètre d'évaluation de qualité vers une gamme de valeurs de qualité prédéfinie ou de maintenir la valeur du paramètre d'évaluation de qualité dans une gamme de valeurs de qualité prédéfinie.

2. Dispositif électronique selon la revendication 1, dans lequel la gamme de valeurs de qualité est définie par un écart déterminé entre le paramètre d'évaluation de la qualité (PEQ) courant et le paramètre d'évaluation de qualité (PEQ) qui serait obtenu avec un calcul en pleine précision.

3. Dispositif électronique selon l'une des revendications précédentes, dans lequel le signal de mesure (x) est un signal électrophysiologique, en particulier un signal électrophysiologique émis par le cortex.

4. Dispositif électronique selon l'une des revendications précédentes, comprenant un circuit intégré qui comprend au moins l'un parmi l'extracteur de caractéristiques (3), le module de contrôle (7), et le décodeur primaire (4).

5. Dispositif électronique selon l'une des revendications précédentes, dans lequel l'actionneur (6) est un élément parmi un stimulateur de moelle épinière, un stimulateur musculaire, un actionneur d'exosquelette, un générateur de parole, un actionneur de fauteuil roulant, tel qu'un moteur de fauteuil roulant, un afficheur.

6. Dispositif électronique selon l'une des revendications précédentes, dans lequel l'extracteur de caractéristique (3) est configuré pour réaliser un calcul approximé d'une convolution entre le signal d'entrée (x) et un signal de référence, en prenant en compte le paramètre de contrôle de précision (PCP).

7. Dispositif électronique selon la revendication précédente, dans lequel le paramètre de contrôle de précision (PCP) correspond à un niveau de précision d'une fonction d'approximation du signal de référence.

8. Dispositif électronique selon l'une des revendications précédentes, dans lequel l'extracteur de caractéristique (3) est configuré pour extraire un ensemble de caractéristiques du signal de mesure, le module de contrôle étant configuré pour déterminer un paramètre de contrôle de précision (PCP) unique pour plusieurs caractéristiques extraites de l'ensemble de caractéristiques, ou pour déterminer un paramètre de contrôle de précision individuel pour chacune des caractéristiques extraites de l'ensemble de caractéristiques.

9. Dispositif électronique selon la revendication précédente, dans lequel le module de contrôle est configuré pour déterminer un paramètre d'évaluation de qualité (PEQ) unique pour plusieurs caractéristiques extraites de l'ensemble de caractéristiques, ou pour déterminer un paramètre d'évaluation de qualité individuel pour chacune des caractéristiques extraites de l'ensemble de caractéristiques.

10. Dispositif électronique selon la revendication précédente, dans lequel la pluralité de caractéristiques extraites (S) correspond à une pluralité de fréquences et/ou une pluralité de localisation spatiales.

11. Dispositif électronique selon l'une des revendications précédentes, comprenant un décodeur secondaire (8) configuré pour fournir un signal de satisfaction d'un utilisateur, le paramètre d'évaluation de qualité (PEQ) prenant en compte le signal de satisfaction de l'utilisateur.

12. Dispositif électronique selon l'une des revendications précédentes, dans lequel le module de contrôle (7) est configuré pour réaliser ou commander périodiquement un calcul d'extraction de caractéristique exact et pour mesurer un écart entre le résultat obtenu avec une extraction de caractéristique exacte et celui obtenu avec une extraction de caractéristique approximée avec le niveau du paramètre de contrôle de précision (PCP) d'extraction courant de façon à déterminer une valeur du paramètre d'évaluation de qualité (PEQ).

13. Dispositif électronique selon l'une des revendications précédentes, dans lequel le module de contrôle (7) est configuré pour déterminer le paramètre d'évaluation de qualité (PEQ) en prenant en compte une valeur d'un indicateur de performance, le dispositif comprenant un capteur de mesure secondaire (9a) configuré pour acquérir une grandeur de contrôle (GC) ou une interface utilisateur (9b) configurée pour collecter une information de contrôle (IC) provenant d'un utilisateur, le module de contrôle (7) étant configuré pour déterminer le paramètre d'évaluation de qualité (PEQ) en prenant en compte l'indicateur de performance déterminé à partir d'une valeur de la grandeur de contrôle (GC) ou de l'information de contrôle (IC).

14. Dispositif électronique selon l'une des revendications précédentes, dans lequel le module de contrôle (7) est configuré pour déterminer le paramètre d'évaluation de qualité (PEQ) en prenant en compte une détection d'anomalie prenant en compte la valeur du signal de commande (C) et la valeur des caractéristiques extraites (S).

15. Dispositif électronique selon l'une des revendications précédentes, dans lequel le module de contrôle (7) est configuré pour déterminer le paramètre d'évaluation de qualité (PEQ) en prenant en compte une valeur d'un niveau de confiance (Cf) ou de probabilité d'un résultat du décodeur primaire (4).

16. Dispositif électronique selon l'une des revendications précédentes, dans lequel le module de contrôle (7) est configuré pour déterminer le paramètre de contrôle de précision de l'extraction en prenant en compte :
- un ensemble de configurations possibles ordonnées du paramètre de contrôle de précision d'extraction ;
- une valeur courante du paramètre de contrôle de précision (PCP) d'extraction choisie parmi l'ensemble de configurations possibles du paramètre de contrôle de précision (PCP) ;
- un incrément dépendant du paramètre de contrôle de qualité (PEQ) d'extraction et/ou de décodage définissant le passage de la valeur courante du paramètre de contrôle de précision (PCP) à une nouvelle valeur du paramètre de contrôle de précision (PCP) parmi l'ensemble de configurations possibles ordonnées du paramètre de contrôle de précision (PCP).

17. Dispositif électronique selon l'une des revendications précédentes, dans lequel le module de contrôle est configuré pour déterminer le paramètre de contrôle de précision (PCP) de l'extraction en prenant en compte :
- une valeur courante du paramètre de contrôle de précision (PCP) d'extraction ;
- une modification du paramètre de contrôle de précision d'extraction, pour un sous-ensemble de caractéristique extraites (S) aléatoire ou prédéfini ou basé sur des heuristiques apprises ou prédéfinies dépendant du paramètre de contrôle de qualité (PEQ).

18. Procédé d'extraction de caractéristiques d'un signal de mesure (x) d'au moins un paramètre physiologique d'un être vivant comprenant :
- Une étape d'acquisition (Et1) du signal de mesure (x) de l'au moins un paramètre physiologique d'un être vivant ;
- Une étape d'extraction (Et2) de caractéristiques (S) du signal de mesure (x) ;
- Une étape de décodage (Et3) utilisant les caractéristiques extraites (S) afin de déterminer un signal de commande (C) d'un actionneur avec lequel l'être vivant interagit ;
- Une étape de pilotage (5) de l'actionneur (6) en fonction du signal de commande (C)
dans lequel l'étape d'extraction (Et2) de caractéristiques du signal de mesure (x) est réalisée de façon approximée en prenant en compte un paramètre de contrôle de précision (PCP), le procédé comprenant en outre :
- Une étape de détermination (Et4) d'un paramètre d'évaluation de qualité (PEQ) ;
- Une étape d'ajustement (Et5) du paramètre de contrôle de précision (PCP) en fonction d'une valeur du paramètre d'évaluation de qualité (PEQ) en choisissant ledit paramètre de contrôle de précision (PCP) correspondant au niveau de précision le plus faible permettant de faire tendre la valeur du paramètre d'évaluation de qualité vers une gamme de valeurs de qualité prédéfinie ou de maintenir la valeur du paramètre d'évaluation de qualité dans une gamme de valeurs de qualité prédéfinie.
